# EUROPEAN PATENT SPECIFICATION

(11) **EP 4 195 994 B1**
(45) Date of publication and mention of the grant of the patent: **22.04.2026**
(21) Application number: 21791536.2
(22) Date of filing: 21.09.2021
(51) Int. Cl.: A61B 1/00, A61B 1/015, B05B 7/24, B05B 9/04

(54) **ENDOSCOPIC SYSTEMS WITH FLUID CONTAINER ADAPTERS**
ENDOSKOPISCHE SYSTEME MIT ADAPTERN FÜR FLÜSSIGKEITSBEHÄLTER
SYSTÈMES ENDOSCOPIQUES AVEC ADAPTATEURS POUR CONTENEURS DE FLUIDES

(30) Priority: 28.09.2020 US 202063084274 P; 28.09.2020 US 202063084284 P; 28.09.2020 US 202063084292 P; 28.09.2020 US 202063084297 P
(43) Date of publication of application: 21.06.2023
(60) Divisional application: 25170524.0
(73) Proprietor: Boston Scientific Scimed, Inc., Maple Grove, MN 55311 (US)
(72) Inventor: POLLOCK, Ryan Vincent William, Maple Grove, Minnesota 55311 (US); SMITH, Paul, Maple Grove, Minnesota 55311 (US); VILLARREAL, Carolina, Maple Grove, Minnesota 55311 (US)
(74) Representative: Vossius & Partner Patentanwälte Rechtsanwälte mbB
(86) International application number: PCT/US2021/051312
(87) International publication number: WO 2022/066643

(56) References cited:
- WO-A1-2014/043765
- CN-U- 210 330 526
- US-A1- 2002 079 280
- US-A1- 2012 277 536
- US-A1- 2019 117 046
- US-B1- 6 354 449

## Description

### FIELD

The present disclosure relates generally to adapters for fluid containers. In particular, the present disclosure relates to universal fluid container adapters for endoscopic systems.

### BACKGROUND

Endoscopy procedures that use typical endoscopes have some common functionalities available to an operator. One includes the ability to insufflate a patient by passing a fluid, such as air or carbon dioxide, through the endoscope in a controlled manner into a target luminal space. Another includes the ability to flush water across the imaging lens to clear the field of view. Yet another of the common functionalities includes the ability to irrigate the lumen to clean surfaces and aid in flushing/suctioning debris during a procedure. Oftentimes, these common functionalities, among others, are driven during a procedure by one or more fluid containers and/or sources. For example, an air pump or carbon dioxide source for insufflation, a water bottle for lens cleaning, and/or a sterile water bottle for irrigation. In some cases, a hybrid tubing set may be used to drive both lens cleaning and irrigation from a sterile water bottle. In either case, the one or more fluid containers and/or sources must be attached to the tubing set of the endoscope. It is with all of the above considerations in mind that the improvements of the present disclosure may be useful.

US 2002/079280 A1 discloses a container made from a vial-like mass produced bottle with a structurally detailed, separately molded plastic neck mounted thereon. The open end of the bottle has a neck with a flange forming an annular shoulder. A plastic neck insert is provided that has a threaded neck opposite a resilient sleeve. The resilient sleeve is adapted to expand to receive the flange in a snap fit type engagement. An aluminum ferrule is press fit over the resilient sleeve of the plastic neck insert to lock the plastic neck insert onto the flange. The ferrule has an upwardly directed edge that engages a downwardly directed edge of the sleeve in interference fit to lock the ferrule onto the sleeve. An elastic seal is provided between the plastic neck insert and the bottle.

### SUMMARY

The invention is defined in claim 1. Further aspects and preferred embodiments are defined in the appended claims.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
**FIG. 1** illustrates a fluid container adapter in conjunction with a neck of a fluid container according to one or more embodiments disclosed hereby.
**FIG. 2** illustrates a fluid container adapter in conjunction with a fluid container according to one or more embodiments disclosed hereby.
**FIGS. 3A** and **3B** illustrate various aspects of a fluid container adapter in conjunction with a fluid container according to one or more embodiments disclosed hereby.
**FIG. 4** illustrates a fluid container adapter in conjunction with a fluid container according to one or more embodiments disclosed hereby.
**FIGS. 5A-5F** illustrate various aspects of a fluid container adapter in conjunction with a fluid container according to one or more embodiments disclosed hereby.

### DETAILED DESCRIPTION

Various embodiments are generally directed to fluid container adapters that couple with a fluid container, so to facilitate access to the contents of the fluid container, such as by an endoscopic system via a tubing set. Several embodiments are particularly directed to fluid container adapters able to couple with a variety of fluid containers to place one or more tubes in fluid communication with an interior of any particular one of the variety of fluid containers. In one embodiment, for example, a fluid container adapter may include a shell with an interior cavity configured to couple with a fluid container. In such embodiments, the fluid container adapter may include an elastic member disposed in the interior cavity of the shell. The elastic member may include a cylindrical shape and be configured to interface with an exterior portion of the fluid container. These and other embodiments are described and claimed.

Some challenges in coupling with a fluid container and gaining access to the contents of the fluid container include having a fluid container adapter that is compatible with the fluid container. For example, a fluid container adapter may include a screw cap with one or more tubes extending therethrough. In such examples, the screw cap may couple to corresponding static threads on a neck of the fluid container with the one or more tubes extending therethrough enabling the endoscopic system to access the contents of the fluid container. However, there are many different types of fluid container manufacturers that offer different fluid container designs. Further, manufacturers may offer different fluid container designs and/or periodically change or update fluid container designs. For instance, manufacturers may offer designs with different thread patterns around the world based on regional preferences or demands. This presents a challenge for manufacturers of tubing sets by requiring them to offer multiple products with customized fluid container adapters for each design. Further, product acquisition and stocking by health care facilities is complicated by necessitating they ensure that tubing sets have a fluid container adapter that is compatible with an available fluid container.

Accordingly, various embodiments of the present disclosure include fluid container adapters that widen the scope of compatibility to a variety of different fluid container designs. In many embodiments, one or more fluid container adapters of the present disclosure may provide an efficient, safe, and effective way to couple with and gain access to the contents of a multitude of fluid container designs. Enabling fluid container adapters to be compatible with different fluid container designs allows medical device companies (e.g., manufacturers of tubing sets) to offer products that are more adaptable and appeal to a broader market. Further, enabling fluid container adapters to be compatible with different fluid container designs can simplify product acquisition and stocking by health care facilities.

It may be understood that the disclosure included herein is exemplary and explanatory only and is not restrictive. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." Although endoscopes and endoscopic systems are referenced herein, reference to endoscopes, endoscopic systems, or endoscopy should not be construed as limiting the possible applications of the disclosed aspects. The invention relates to a system for use with a medical device. For example, the disclosed aspects may be used in conjunction with duodenoscopes, bronchoscopes, ureteroscopes, colonoscopes, catheters, diagnostic or therapeutic tools or devices, or other types of medical devices or systems.

Reference is now made to the drawings, wherein like reference numerals are used to refer to like elements throughout. In the following description, for purpose of explanation, numerous specific details are set forth in order to provide a thorough understanding thereof. It may be evident, however, that the novel embodiments can be practiced without these specific details. In other instances, well known structures and devices are shown in block diagram form to facilitate a description thereof. The intention is to cover all modification, equivalents, and alternatives within the scope of the claims.

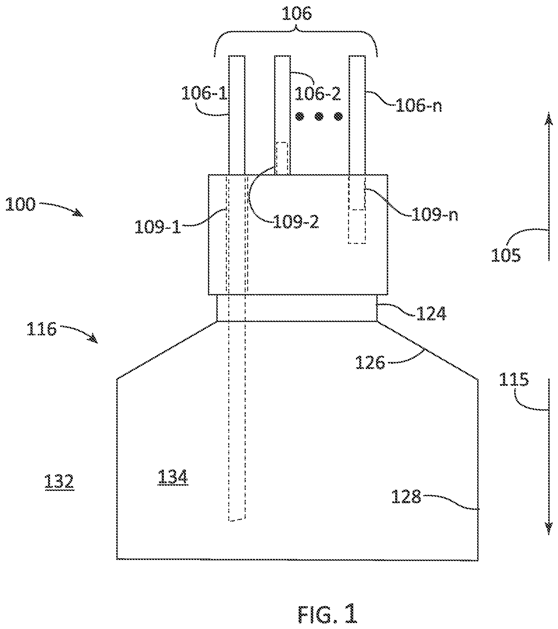

**FIG. 1** illustrates a fluid container adapter 100 in conjunction with a tube set 106 and a fluid container 116 according to one or more embodiments of the present disclosure. In one or more embodiments of the present disclosure, components of the fluid container adapters may interoperate to couple with a fluid container and facilitate access to the contents of the fluid container via a tube set, such as a tube set for an endoscopic system. In the illustrated embodiment, fluid container adapter 100 couples to fluid container 116 and places a tube set 106 of one or more tubes 106-1, 106-2, 106-n in fluid communication with an interior 134 of the fluid container 116. Further, at least a portion of each tube in set 106 is on an exterior 132 of the fluid container 116. In several embodiments, fluid container adapter 100 may couple to and/or seal with a portion of the fluid container 116. The components of FIG. 1 are oriented with a top 105 and a bottom 115. In some embodiments, FIG. 1 may include one or more components that are the same or similar to one or more other components of the present disclosure. Further, one or more components of FIG. 1, or aspects thereof, may be incorporated into other embodiments of the present disclosure without departing from the scope of this disclosure. Embodiments are not limited in this context.

Various embodiments disclosed hereby are directed to fluid container adapters that couple with a fluid container to enable access to the contents of the fluid container, such as from an endoscopic system via a tubing set. Several embodiments are particularly directed to a fluid container adapter that can couple with an opening in a variety of fluid containers to place one or more tubes in fluid communication with an interior of any particular one of the variety of fluid containers. In one or more embodiments, the fluid container adapter 100 may enable tube set 106 to be used in conjunction with a plurality of types and designs of fluid containers. The fluid container adapter 100 may include one or more passages 109-1, 109-2, 109-n to enable tubes to be placed in fluid communication with the interior 134 of the fluid container 116.

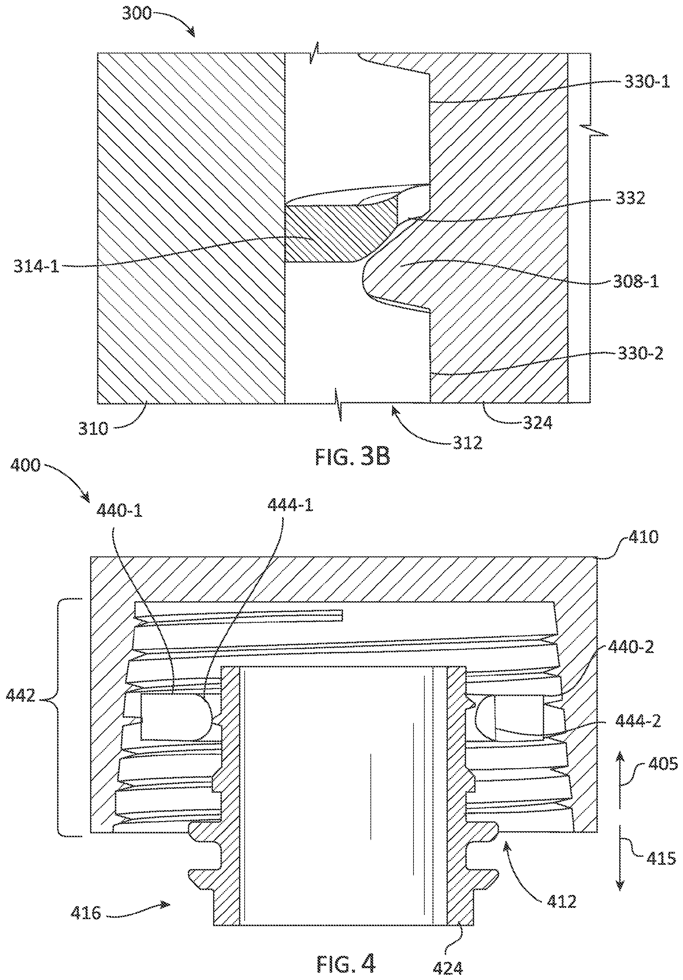

Generally, the fluid container 116 includes a neck 124, a shoulder 126, and a body 128. Additionally, the fluid container 116 may include an opening, such as at the top of the neck 124 (see e.g., FIG. 4). In various embodiments, the fluid container adapter 100 may couple to one or more portions of the fluid container 116, such as the neck 124. In various such embodiments, the fluid container adapter 100 may block and/or seal the opening in the fluid container 116. The fluid container adapter 100 may couple to and/or seal with interior and/or exterior portions of the fluid container 116. For example, a portion of the fluid container adapter 100 may be inserted into the fluid container 116.

The fluid container adapter 100 may be constructed from a variety of materials and or components. For instance, the fluid container adapter 100 may include portions constructed from one or more materials having one or more characteristics, such as flexible, rigid, semi-rigid, semi-flexible, deformable, conformable, sealing, shape-memory, elastomeric, polymeric, and the like. In some embodiments, one or more of the materials may deform permanently. For example, the fluid container adapter 100 may include a permanently deformable material that creates a permanent female version of the first fluid container threaded into the fluid container adapter 100. In other embodiments, the deformation may be nonpermanent. In many embodiments, the fluid container adapter 100 may include layers of one or more materials. For example, the fluid container adapter 100 may include a skeleton layer of rigid materials and a skin layer of sealing material.

As previously mentioned, the fluid container adapter 100 may include one or more passages 109-1, 109-2, 109-n to enable tubes to be placed in fluid communication with the interior 134 of the fluid container 116. The passages 109 may place tubes in fluid communication with the interior 134 of the fluid container 116 in a variety of manners. For example, passage 109-1 may extend from the top to the bottom of the fluid container adapter 100. In another example, passage 109-2 may include a protrusion, such as with one or more Luer lock barbs, that tube 106-2 couples to. In yet another example, passage 109-n may extend through a portion of the fluid container adapter 100.

In some embodiments, one or more of the passages 109 may couple a tube in tube set 106 with another tubular member. In some such embodiments, the other tubular member may extend from the bottom of the passage. For example, the other tubular member may include a straw coupled to the bottom end of passage 109-n. In various embodiments, the other tubular member may include different characteristics than the tube in tube set 106. The other tubular member may be configured for interfacing with contents in a fluid container. For example, the other tubular member may be more flexible that the tube in tube set 106 to facilitate efficient movement of a pickup (e.g., orifice for drawing/expelling in/out fluids from the tube) to the lowest point in the fluid container. In another example, the other tubular member may be more rigid than the tube in tube set 106 to prevent kinking or curling.

In several embodiments, the fluid container adapter 100 may create a seal with one or more tubes in the tube set 106, such as by extending a tube through a passage or coupling the tube with the passage. For instance, passage 109-1 may include a lining that creates a slidable seal with tube 106-1. In some embodiments, passage 109-1 may form a fluid tight seal with the exterior of tube 106-1, the protrusion of passage 109-2 may form a fluid tight seal with the interior of tube 106-2, and passage 109-n may form a fluid tight seal with the exterior of tube 106-n. In some such embodiments, the fluid tight seals may be maintained as one or more of the tubes 106-1, 106-n are slid up and down in passages 109-1, 109-n, respectively.

Accordingly, in some embodiments, the fluid container adapter 100 may place the tube set 106 in fluid communication with the interior 134 of the fluid container 116 while keeping the interior 134 of the fluid container 116 sealed from the exterior 132. In some embodiments, the fluid container adapter 100 may maintain a slidable seal with the exterior of one or more tubes in tube set 106. For instance, the fluid container adapter 100 may maintain a fluid seal with the exterior of a tube as the tube is inserted through the fluid container adapter 100 and into the interior 134 of the fluid container 116.

In various embodiments, the tube set 106 may be used to remove fluids from and/or introduce fluids into the interior 134 of the fluid container 116. For example, tube 106-2 may be used to pump a gas (e.g., air, carbon dioxide, etc.) into the fluid container 116 and tube 106-1 may allow a liquid (e.g., water, saline, etc.) to exit the fluid container 116 as a result of the gas being pumped into the fluid container 116. In another example, tube 106-1 may be used to suck a liquid out of the fluid container 116 and tube 106-2 (or a valve built into the fluid container adapter 100) may allow gas pressure in the fluid container 116 to equalize with the atmosphere. In such other examples, a one-way valve and/or a pressure relief valve may be utilized to equalize with the atmosphere. In various embodiments, one or more of the tubes in tube set 106 may comprise an irrigation line for an endoscopic system.

As shown in the illustrated embodiment, the tubes 106-1, 106-2, 106-n may extend a variety of lengths into the fluid container 116. In many embodiments, the length of each of the tubes may be determined based on the function of the tube. For instance, on one hand, a vent tube may end proximate to the top 105 or couple to a passage with a fitting protruding on the exterior 132 of the fluid container adapter 100. On the other hand, a tube for removing contents, such as a liquid, from the fluid container 116 may extend to the bottom 115 of the fluid container 116. Oftentimes, a first tube of the tube set 106 may be placed in fluid communication with a first fluid (e.g., a gas) in the interior 134 of the fluid container 116 and a second tube of the tube set 106 may be placed in fluid communication with a second fluid (e.g., a liquid) in the interior 134 of the fluid container 16.

In one or more embodiments, fluid container adapter 100 may include one or more valves (e.g., one-way vales, pressure relief vales, two-way valves, and the like) to control the flow of fluids into and/or out of the fluid container 116. For example, a one-way valve may be utilized to prevent back flow into fluid container 116. Further, in some embodiments, one or more of the tubes may be coaxial. For example, a tube may be disposed coaxially with a second tube member having a larger diameter, resulting in inner and outer tubes. In some such examples, the inner and outer tubes may extend through a single passage in the fluid container adapter 100.

For this and other reasons, in many embodiments, the number of passages 109 may not directly correspond to the number of tubes in tube set 106. For example, different endoscopic systems may require different numbers of tubes in fluid communication with the interior of a fluid container. Accordingly, one or more passages may be blocked or unblocked (e.g., with a plug or cap) and/or one or more tubes may be removed or added to accommodate different endoscopic systems or different configuration of an endoscopic system. In other words, fluid container adapters can be adapted to different tube configurations and/or tube sets, such as via a variety of types of passages (e.g., passages 109-1, 109-2, 109-n).

The components of fluid container adapters disclosed hereby may be constructed from a variety of material types and compositions including one or more of polymers, elastomers, metals, alloys, shape-memory materials, rubber, silicon, plastic, composite materials, ceramics, polycarbonate, acrylonitrile butadiene styrene (ABS), high-density polyethylene (HDPE), Nylon, polyether ether ketone (PEEK), thermoplastic, stainless steel, titanium, aluminum, and the like. In some embodiments, different portions and/or components may include one or more different types, compositions, or structures of materials. For example, a shell of the fluid container adapter may be constructed from a first type of polymer and an elastic member of the fluid container adapter may be constructed from a second type of polymer with a lower durometer than the first type of polymer. In some embodiments, one or more components and/or portions of the fluid container adapter may include multiple material layers and/or embeddings, such as a semi-rigid skeletal structure.

An exemplary method for utilizing one or more fluid container adapters disclosed hereby (e.g., fluid container adapter 100) may proceed with one or more of the following steps. A first end of one or more tubes may be connected to an endoscopic device or system and a second end of the one or more tubes may be coupled to a protrusion and/or extended through one or more passages in the fluid container adapter. The fluid container adapter may be coupled to a fluid container to place one or more of the tubes in fluid communication with the interior of the fluid container. In several embodiments, the fluid container adapter may seal the interior of the tubes and/or fluid container from an exterior environment. In many embodiments, the fluid container adapter may couple to a fluid container proximate an opening in the fluid container (e.g., opening in a fluid container when a cap is removed). In many such embodiments, one or more of the tubes may extend into the fluid container via the opening. In some embodiments, the fluid container adapter may attach to, cover up, or seal with the opening in the fluid container. One or more fluids may be introduced into and/or removed from the interior of the fluid container via a tube set when the fluid container adapter is coupled to the fluid container.

An exemplary method for manufacturing one or more fluid container adapters disclosed hereby (e.g., fluid container adapter 100) may proceed with one or more of the following steps. Forming a shell with an interior cavity and one or more holes extending through the shell and into the interior cavity; and disposing an elastic member in the interior cavity, the elastic member comprising a cylindrical shape and configured to seal with an exterior portion of the fluid container. In some embodiments, the elastic member is disposed in the interior cavity via overmolding.

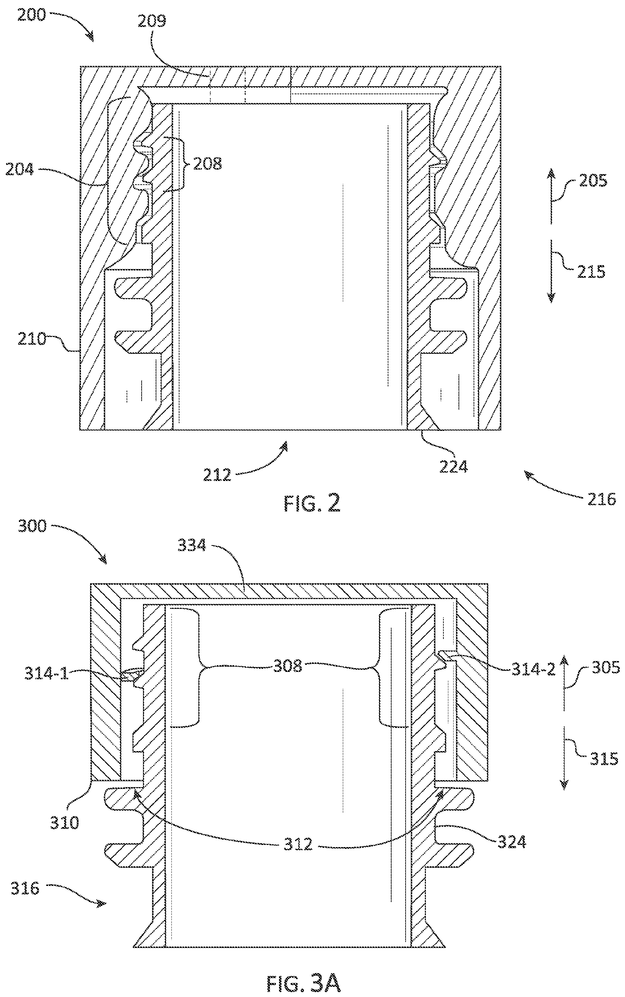

**FIG. 2** illustrates fluid container adapter 200 in conjunction with a fluid container 216 according to one or more embodiments of the present disclosure. In the illustrated embodiment, fluid container adapter 200 couples to a neck 224 of the fluid container 216 to place one or more tubes in fluid communication with an interior of the fluid container, such as by extending the one or more tubes through passage 209. The fluid container adapter 200 may utilize an elastic member 204 to couple with the neck 224 of the fluid container. In some embodiments, FIG. 2 may include one or more components that are the same or similar to one or more other components of the present disclosure. Further, one or more components of FIG. 2, or aspects thereof, may be incorporated into other embodiments of the present disclosure without departing from the scope of this disclosure. For example, elastic member 204 may be incorporated into fluid container adapter 100 without departing from the scope of this disclosure. The components of FIG. 2 are oriented with a top 205 and a bottom 215. Embodiments are not limited in this context.

Fluid container adapter 200 includes a shell 210 with a passage 209 and an interior cavity 212. The shell may comprise one or more of a plastic and a metal. In the illustrated embodiment, a majority of the interior cavity 212 is filled with the neck 224 of fluid container 216. In various embodiments, the passage 209 may be configured to receive, or have extended therethrough, a tube being placed in fluid communication with the interior of the fluid container. Passage 209 may be the same or similar to one or more of passages disclosed hereby, such as passages 109-1, 190-2, 109-n. The fluid container may include neck 224 with threaded portion 208.

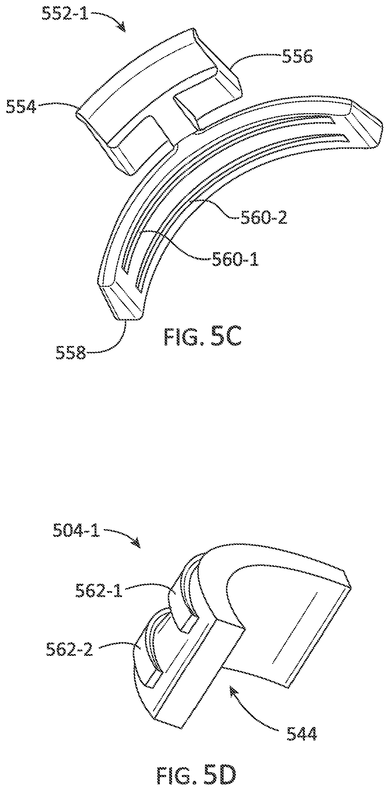

In one or more embodiments, the elastic member 204 may be disposed within the interior cavity 212 of the shell 210. The elastic member 204 may comprise one or more of rubber, silicone, an elastomer, or shape memory material. In some embodiments, the elastic member may be disposed within the interior cavity 212 via overmolding. In other embodiments, the elastic member 204 may be disposed within the interior cavity 212 via a snap fit between corresponding features (see e.g., channels 560 and channel members 562 in FIGS. 5C-5F). The elastic member 204 may be configured to interface with an exterior portion of the fluid container 216. For example, the elastic member 204 may be configured to couple with, conform to, and/or seal with the threaded portion 208 of neck 224. On the other hand, shell 210 may be rigid relative to the elastic member. In several embodiments, the shell 210 may provide a rigid support structure for the elastic member 204. As shown in the illustrated embodiment, the elastic member 204 may couple with, conform to, and seal with male threads included in the threaded portion 208 of the neck 224. In several embodiments, the elastic member 204 may include a cylindrical shape. In one or more embodiments, the elastic member 204 may include a shape-memory material, such as a shape-memory elastomer. In various embodiments, the elastic member 204 may include a cylindrical shape with a smooth interior surface. In other embodiments, the elastic member 204 may include a cylindrical shape with an interior surface having one or more indentations and/or protrusions. For example, the elastic member 204 may include indentations comprising starter threads.

**FIGS. 3A** and **3B** illustrate various aspects of a fluid container adapter 300 in conjunction with a fluid container 316 according to one or more embodiments of the present disclosure. More specifically, FIG. 3A illustrates fluid container adapter 300 with adjustable threads 314-1, 314-2 (or adjustable threads 314) in conjunction with fluid container 316 and FIG. 3B illustrates a detail view of adjustable thread 314-1 interfacing with the neck 324 of fluid container 316. In some embodiments, FIGS. 3A and/or 3B may include one or more components that are the same or similar to one or more other components of the present disclosure. Further, one or more components of FIGS. 3A and/or 3B, or aspects thereof, may be incorporated into other embodiments of the present disclosure without departing from the scope of this disclosure. For example, shell 310 may be the same or similar to the shell 210. The components of FIGS. 3A and 3B are oriented with a top 305 and a bottom 315. Embodiments are not limited in this context.

In the illustrated embodiment, fluid container adapter 300 may include a shell 310 with a flat-planar surface 334 and adjustable threads 314-1, 314-2. It will be appreciated that adjustable threads 314-1, 314-2 may be part of a continuous spiral adjustable thread. In various embodiments, the adjustable threads 314 may conform to a threaded portion 308 on the neck 324 of fluid container 316. More generally, fluid container adapter 300 may include one or more adjustable threads that can couple with, conform to, and/or seal with a variety of different thread types may be disposed, such as via overmolding, in the interior cavity 312 of the shell 310. For instance, when the adjustable threads 314 encounter a difference in pitch and/or different outside diameter, the adjustable threads 314 may deform to adapt to the threads of the fluid container. In many embodiments, the adjustable threads 314 may be able to accommodate a broader range of male threads on a fluid container than the elastic member 204 . On the other hand, the elastic member 204 may accommodate a broader range of female threads than adjustable threads 314. In some embodiments, adjustable threads 314 may include or be referred to as an elastic member.

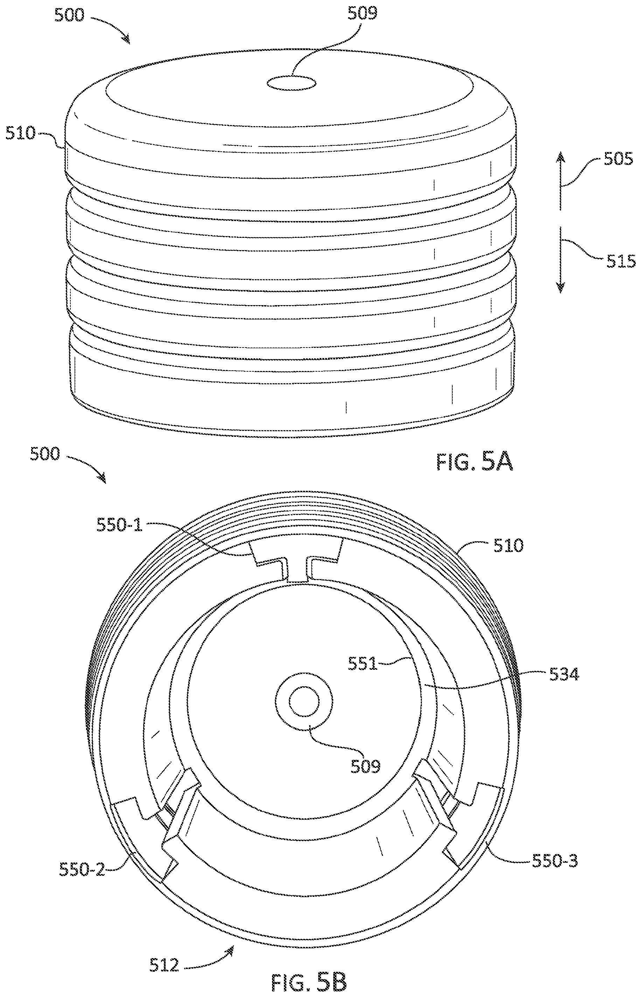

The flat-planar surface 334 of shell 310 may comprise a top of the fluid container adapter 300. In several embodiments, the flat-planar surface 334 may include one or more passages. The flat-planar surface 334 may be a circular member (e.g., disc) and/or include a circular portion. In some embodiments, an elastic disc may be disposed on the flat-planar surface 334 in the interior cavity 312. In some such embodiments, the elastic disc may contact and/or seal with a top exterior surface of the neck 324 (see e.g., elastic disc 551 of FIG. 5B).

In one or more embodiments, the adjustable threads 314 may be disposed within the interior cavity 312 of the shell 310. The adjustable threads 314 may comprise one or more of rubber, silicone, an elastomer, or a shape memory material. In various embodiments, the adjustable threads 314 may be disposed within the interior cavity 312 via overmolding. In other embodiments, the adjustable threads 314 may be disposed within the interior cavity 312 via a snap fit between corresponding features (see e.g., channels 560 and channel members 562 in FIGS. 5C-5F). The adjustable threads 314 may be configured to interface with an exterior portion of the fluid container 316. For example, the adjustable threads 314 may be configured to couple with, conform to, and/or seal with the threaded portion 308 of neck 324. As shown in the illustrated embodiment, the adjustable threads 314 may couple with, conform to, and seal with male threads included in the threaded portion 308 of the neck 324. In several embodiments, the adjustable threads 314 may include one or more of a cylindrical, helical, circular, and curved shape. In one or more embodiments, the adjustable threads 314 may include a shape-memory material, such as a shape-memory elastomer. As shown in the illustrated embodiment, in some embodiments, the adjustable threads 314 may include a tapered portion on the bottom side. In other embodiments, the tapered portion may result from deformation of the adjustable threads 314 due to interfacing with threaded portion 308.

Referring to FIG. 3B, the interior cavity 312 of fluid container adapter 300 may include gaps 330-1, 330-2 above and below the adjustable thread 314-1. When the adjustable thread 314-1 is interfaced with thread 308-1, the adjustable thread 314-1 may deform and/or flex. Further, the adjustable thread 314-1 is configured to displace into at least a portion of the gap 330-1 when interfaced with an exterior portion of the fluid container 316 (e.g., thread 308-1). In many embodiments, the adjustable thread 314-1 may spiral around the interior cavity 312. In some embodiments, another adjustable thread may be disposed below thread 308-1. For example, thread 308-1 may be sandwiched between an upper adjustable thread (e.g., adjustable thread 314-1) and a lower adjustable thread. In some such examples, the upper and lower adjustable threads may comprise a deformable female thread.

Adjustable thread 314-1 may include portions of differing flexibility. In some embodiments, differing flexibility may result from varying a thickness of the adjustable thread. For example, a taper 332 at the end of the adjustable thread 314-1 may be more flexible than other portions of the adjustable thread 314-1 In one or more embodiments, the varying flexibility of the adjustable thread 314-1 may facilitate the fluid container adapter 300 being used with a variety of types of fluid containers.

**FIG. 4** illustrates fluid container adapter 400 in conjunction with a fluid container 416 according to one or more embodiments of the present disclosure. In the illustrated embodiment, fluid container adapter 400 couples to a neck 424 of the fluid container 416 over opening 422 to place one or more tubes in fluid communication with an interior of the fluid container via one or more passages (not shown) in shell 410. The fluid container adapter 400 may utilize one or more carriages (e.g., carriages 440-1, 440-2) moving along a tapered thread 442 to couple with the neck 424 of the fluid container 416. In some embodiments, FIG. 4 may include one or more components that are the same or similar to one or more other components of the present disclosure. Further, one or more components of FIG. 4, or aspects thereof, may be incorporated into other embodiments of the present disclosure without departing from the scope of this disclosure. For example, shell 410 may be incorporated into fluid container adapter 100 without departing from the scope of this disclosure. The components of FIG. 4 are oriented with a top 405 and a bottom 415. Embodiments are not limited in this context.

Fluid container adapter 400 includes shell 410 with an interior cavity 412 having a tapered thread 442 and carriages 440-1, 440-2 with gripping surfaces 444-1, 444-2, respectively. In various embodiments, friction between the gripping surfaces 444 and features of the neck 424 (e.g., male threads) may cause the carriages 440 to advance toward the top of the tapered thread 442 in response to rotation in a first direction. As the carriages 440 advance toward the top of the tapered thread 442, the radial force exerted by the gripping surfaces 444 on the neck 424 may increase. In this way, fluid container adapter 400 may couple with, conform to, and/or seal with fluid container 416.

Similarly, rotation in a second direction may cause the carriages 440 to move toward the bottom of the tapered thread 442, and as the carriages 440 move towards the bottom of the tapered thread 442, the radial force exerted by the gripping surfaces 444 on the neck 424 may decrease, and eventually allow removal of the fluid container adapter 400 from fluid container 416. In some embodiments, the gripping surfaces 444 may be disposed on the carriages 440, respectively, via overmolding. In other embodiments, , the gripping surfaces 444-1, 444-2 may be disposed on the carriages 440-1, 440-2, respectively, via a snap fit between corresponding features (see e.g., channels 560 and channel members 562 in FIGS. 5C-5F).

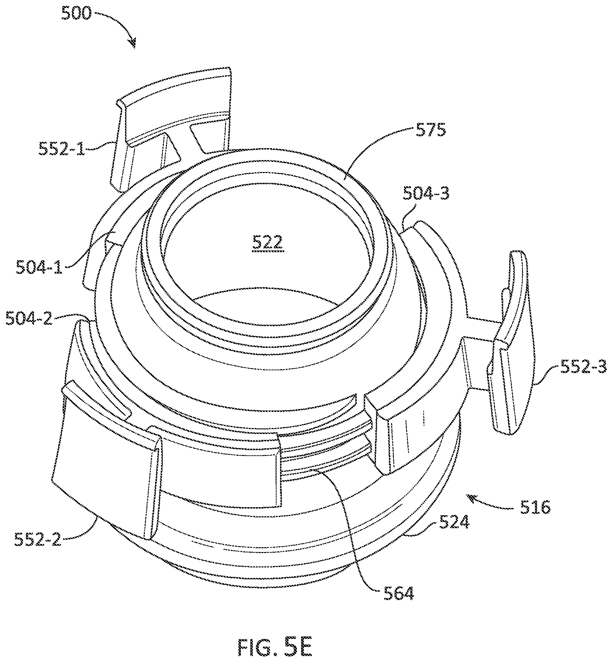

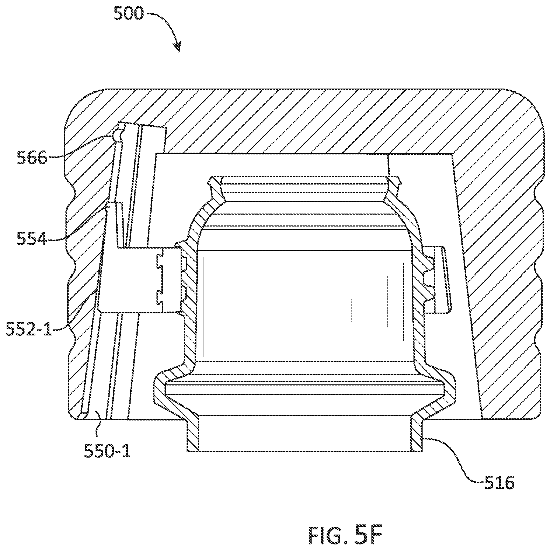

**FIGS. 5A-5F** illustrate various aspects of a fluid container adapter 500 in conjunction with a fluid container 516 according to one or more embodiments of the present disclosure. More specifically, FIG. 5A illustrates a side perspective view of a shell 510 of the fluid container adapter 500. FIG. 5B illustrates a bottom perspective view of the shell 510 of the fluid container adapter 500. FIG. 5C illustrates a perspective view of a carriage of the fluid container adapter 500. FIG. 5D illustrates an elastic member 504-1 of the fluid container adapter 500. FIG. 5E illustrates a perspective view of the fluid container adapter 500 with the shell 510 removed and in conjunction with fluid container 516. FIG. 5F illustrates a cross-sectional view of the fluid container adapter 500 in conjunction with the fluid container 516. In some embodiments, FIGS. 5A-5F may include one or more components that are the same or similar to one or more other components of the present disclosure. Further, one or more components of FIGS. 5A-5F, or aspects thereof, may be incorporated into other embodiments of the present disclosure without departing from the scope of this disclosure. For example, carriages 552 may be the same or similar to carriages 440. In another example, one or more of passages 109-1, 109-2, 109-n may be incorporated into fluid container adapter 500 without departing from the scope of this disclosure. The components of FIGS. 5A-5F are oriented with a top 505 and a bottom 515. Embodiments are not limited in this context.

Referring to FIG. 5A, the fluid container adapter 500 may include a shell 510 with a passage 509. Oftentimes, the shell 510 may include one or more ergonomic features. For example, shell 510 may include one or more surface features (e.g., longitudinal channels in shell 510) to improve user experience. In various embodiments, the surface features may include, contours, protrusions, channels, ridges, bumps, hatching, and the like.

Referring to FIG. 5B, the shell 510 may include an opening of passage 509 in flat-planar surface 534, a plurality of slides 550-1, 550-2, 550-3, and an elastic disc 551 disposed on the flat-planar surface. The passage 509 may extend from an exterior surface of the shell 510 to an interior surface of the shell 510 (e.g., inside the interior cavity 512). In some embodiments, the elastic disc 551 may be disposed on the flat-planar surface 534 in the interior cavity 512. In some such embodiments, the elastic disc may contact and/or seal with a top exterior surface of the fluid container 516 (see e.g., exterior surface 575 of FIG. 5E). The slides 550 may comprise recessed channels in the wall of the interior cavity 512. As will be discussed in more detail below, carriages may move along the slides 550 similar to the carriages 440 of fluid container adapter 400 moving along the tapered thread 442. The slides 550 may be offset from each other. For example, in the illustrated embodiment, the slides 550 are offset from each other by 120 degrees. The interior cavity 512 may have a taper. For instance, the diameter may decrease towards the top of the shell 510.

In FIG. 5C, a carriage 552-1 is illustrated. The carriage 552-1 may move along one of the slides 550 with a portion of the carriage 552-1 being disposed therein. The carriage 552-1 may include a guide shoe 556 with a detent 554 and a mount 558 with channels 560-1, 560-2. The guide shoe 556 may be disposed in one of the slides 550. The taper of the interior cavity 512 may cause the carriage 552-1 to move towards a longitudinal axis at the center of the interior cavity 512 when the carriage 552-1 is moved along the slide 550-1 towards the top of the shell 510. The guide shoe 556 may include a shape that corresponds to a slide. In some embodiments, the corresponding shapes between the guide shoe 556 and the slide may be configured to restrict the carriage 552-1 to linear movement. As will be described in more detail below, the detent 554 may be configured to retain the carriage 552-1 in a predetermined position along one of the slides 550. In some embodiments, retaining the carriage 552-1 in a predetermined position along one of the slides 550 may prevent the fluid container adapter 500 from being used more than once.

In FIG. 5D, an elastic member 504-1 is illustrated. The elastic member 504-1 includes channel members 562-1, 562-2 and gripping surface 544. In some embodiments, gripping surface 544 may be the same or similar to gripping surface 444-1, 444-2. The elastic member 504-1 may be disposed on the carriage 552-1, such as via a snap fit. For example, channel members 562-1, 562-2 of elastic member 504-1 may be received by channels 560-1, 560-2 of carriage 552-1.

Fluid container adapter 500 may include one or more carriages with one or more elastic members. In many embodiments each of the one or more carriages and/or each of the one or more elastic members may be the same or similar. For example, carriage 552-1 may be the same as carriages 552-2, 552-3. In the illustrated embodiment of FIG. 5E, fluid container adapter 500 includes three carriages 552-1, 552-2, 552-3, each with an elastic member 504-1, 504-2, 504-3. The gripping surfaces of the elastic members 504 contact the neck 524 of fluid container 516. Similar to fluid container adapter 400, as carriages move up along respective slides, the radial force of the elastic members 504 on the fluid container 516 increases due to the taper of the interior cavity 512. As shown in FIG. 5E, the elastic members 504-3 contact male threads 564 on the exterior of fluid container 516. Some embodiments may include an interface that allows carriages to be manually positioned. For example, a lever may extend through the shell and enable manual movement of carriages. In many embodiments, the ability to manually adjust the position of carriages may facilitate use of the fluid container adapter with a broader range of fluid container designs (e.g., a larger range of outside diameters).

FIG. 5F illustrates a cross-sectional view of fluid container adapter 500, including the taper of the interior cavity 512, in conjunction with fluid container 516. Further, FIG. 5F includes slide 550-1 with catch 566 and carriage 552-1 with detent 554. As previously mentioned, the detent 554 may be configured to retain the carriage 552-1 in a predetermined position along one of the slides 550. In some embodiments, retaining the carriage 552-1 in a predetermined position along one of the slides 550 may prevent the fluid container adapter 500 from being used multiple times. Accordingly, when carriage 552-1 moves far enough towards the top of slide 550-1, detent 554 may be received by catch 566. In such embodiments, when detent 554 is received by catch 566, the carriage 552-1 may be prevented from returning to the bottom of slide 550-1. In other such embodiments, the detent 554 being received by the catch 566 may reversibly lock the fluid container adapter 500 onto the fluid container 516. In various embodiments, the detent 554 and catch 566 may provide haptic feedback, such as to indicate when the carriage 552-1 cannot move further upwards.

As previously mentioned, various embodiments of the present disclosure include fluid container enclosures that widen the scope of compatibility to a variety of different fluid container designs and/or functionality types, such as insufflation and irrigation. In many embodiments, one or more fluid container enclosures of the present disclosure may provide an efficient, safe, and effective way to couple with and gain access to the contents of a multitude of fluid container designs. Enabling fluid container enclosures to be compatible with different fluid container designs allows manufacturers of tubing sets to offer products that are more adaptable. Further, enabling fluid container enclosures to be compatible with different fluid container designs can simplify product acquisition and stocking by health care facilities.

For example, enclosure systems according to some embodiments may be used with a wide variety of fluid container port, opening, or neck dimensions, such as an opening diameter and/or thread configuration (for instance, Glass Packaging Institute (GPI) thread finish, "H" dimension, thread distance, thread dimensions, and/or the like), thread pitch, outer diameter (OD) of port or neck (OD port), OD of threads (OD thread), thread width, and/or the like. In addition, enclosure systems according to some embodiments may be used on fluid containers made by various manufacturers.

For example, in some embodiments, an enclosure system may include a single cap that is capable of being installed on containers having an opening size (e.g., neck OD, not including threads) of about 10 mm, about 20 mm, about 30 mm, about 40 cm mm about 50 mm, about 100 mm, about 200 mm, and any value or range between any two of these values (including endpoints). In some embodiments, an enclosure system may include a single cap that is capable of being installed on containers having an opening size (e.g., neck OD, not including threads) of about 30 mm, about 30.4 mm, about 31 mm, about 32 mm, about 32.2 mm to about 32.9 mm, about 33 mm, about 33.6 mm, about 34 mm, about 35 mm, about 40 mm, and any value or range between any two of these values (including endpoints).

In some embodiments, an enclosure system may include a single cap that is capable of being installed on containers having a total opening size (e.g., neck OD including threads) of about 10 mm, about 20 mm, about 30 mm, about 40 cm mm about 50 mm, about 100 mm, about 200 mm, and any value or range between any two of these values (including endpoints). In some embodiments, an enclosure system may include a single cap that is capable of being installed on containers having a total opening size (e.g., neck OD including threads) of about 30 mm, about 31 mm, about 32 mm, about 33 mm, about 33.2 mm, about 34 mm, about 35 mm, about 35.75 mm, about 36 mm, about 36.26 mm, about 40 mm, and any value or range between any two of these values (including endpoints).

In some embodiments, an enclosure system may include a single cap that is capable of being installed on containers having a thread width of about 0.5 mm, about 1 mm, about 2 mm, about 3 mm, about 4 mm, about 5 mm, about 10 mm, and any value or range between any two of these values (including endpoints). In some embodiments, an enclosure system may include a single cap that is capable of being installed on containers having a thread width of about 1 mm, about 1.83 mm, about 2 mm, about 2.4 mm, about 2.66 mm, about 3 mm, and any value or range between any two of these values (including endpoints).

In some embodiments, an enclosure system may include a single cap that is capable of being installed on containers having a thread pitch of about 1 mm, about 2 mm, about 3 mm, about 4 mm, about 5 mm, about 6 mm, about 7 mm, about 8 mm, about 9 mm, about 10 mm, and any value or range between any two of these values (including endpoints). In some embodiments, an enclosure system may include a single cap that is capable of being installed on containers having a thread pitch of about 3 mm, about 3.94 mm, about 4 mm, about 4.23 mm, about 4.25 mm, about 5 mm, and any value or range between any two of these values (including endpoints).

In some embodiments, an enclosure system may include a single cap that is capable of being installed on a fluid container having a GPI thread finish of 350, 400, 410, 415, 425, 430, 450, and any value or range between any two of these values (including endpoints).

In various embodiments, an enclosure system may include a single cap that is capable of being installed on different types of fluid container openings or necks. For example, a cap of an enclosure system according to some embodiments may be installed on (and form a seal with) fluid container openings/necks with different dimensions. In some embodiments, an enclosure system may include a single cap that is capable of being installed on a plurality of fluid container necks having a OD thread difference (Δ OD thread) (i.e., the difference between an OD thread of the smallest OD thread neck and the largest OD thread neck) of about 0.25 mm, about 0.5 mm, about 1 mm, about 1.5 mm, about 2 mm, about 3 mm, about 4 mm, about 5 mm, about 10 mm, and any value or range between any two of these values (including endpoints). In various embodiments, an enclosure system may include a single cap that is capable of being installed on a plurality of fluid container openings having a OD opening difference (Δ OD opening) (i.e., the difference between an OD opening of the neck with the smallest OD opening and the port with the largest OD opening) of about 0.25 mm, about 0.5 mm, about 1 mm, about 1.5 mm, about 2 mm, about 3 mm, about 4 mm, about 5 mm, about 10 mm, and any value or range between any two of these values (including endpoints).

All of the devices disclosed and claimed herein can be made and executed without undue experimentation in light of the present disclosure. While the systems of this invention have been described in terms of preferred embodiments, it may be apparent to those of skill in the art that variations can be applied without departing from the scope of the claims. All such equivalents are deemed to be within the scope of the appended claims.

## Claims

1. A system for use with a medical device, the system including:
a fluid container (216, 316, 416, 516), the fluid container (216, 316, 416, 516) including a neck (224, 324, 424, 524) with an exterior threaded portion (208, 308, 408), and
a fluid container adapter (100, 200, 300, 400, 500), comprising:
a shell (210, 310, 410, 510) with an interior cavity (212, 312, 412, 512), wherein the interior cavity is configured to couple with the fluid container (216, 316, 416, 516); and
an elastic member (204, 314-1, 314-2, 440-1, 440-2, 504-1, 504-2, 504-3) disposed in the interior cavity, the elastic member comprising a cylindrical shape and configured to interface with the exterior threaded portion (208, 308, 408) of the fluid container (216, 316, 416, 516).

2. The system of claim 1, wherein the elastic member (204, 314-1, 314-2, 440-1, 440-2, 504-1, 504-2, 504-3) is configured to seal with the exterior portion of the fluid container (216, 316, 416, 516).

3. The system of any of claims 1 to 2, wherein the elastic member (314-1) comprises a first portion with a first thickness and a second portion with a second thickness, wherein the second portion is configured to flex more than the first portion.

4. The system of claim 3, wherein the second thickness is less than the first thickness, and/or wherein the second portion comprises an adjustable thread.

5. The system of any of claims 1 to 4, wherein the shell is more rigid than the elastic member.

6. The system of any of claims 1 to 5, wherein the shell includes one or more through holes (109-1, 109-2, 209, 509).

7. The system of claim 6, wherein the one or more through holes (109-1, 109-2, 209, 509) are configured to place a tube set (106) in fluid communication with an interior portion of the fluid container.

8. The system of any of claims 1 to 7, comprising a gap between the elastic member (204, 314-1, 314-2, 440-1, 440-2, 504-1, 504-2, 504-3) and a portion of the shell (210, 310, 410, 510), the elastic member configured to displace into at least a portion of the gap when interfaced with the exterior portion of the fluid container.

9. The system of any of claims 1 to 8, wherein the elastic member (204, 314-1, 314-2, 440-1, 440-2, 504-1, 504-2, 504-3) comprises a shape memory elastomer.

10. The system of any of claims 1 to 9, wherein the elastic member (204, 314-1, 314-2, 440-1, 440-2, 504-1, 504-2, 504-3) is disposed in the interior cavity (212, 312, 412, 512) via overmolding.

11. The system of any of claims 1 to 10, wherein the shell (510) comprises one or more slides (550-1, 550-2, 550-3) with at least one carriage (552-1, 552-2, 552-3) disposed in each of the one or more slides (550-1, 550-2, 550-3).

12. The system of claim 11, wherein at least a portion of the elastic member is disposed on each of the one or more carriages (552-1, 552-2, 552-3).

13. The system of claim any of claims 1 to 12, wherein the elastic member (204, 314-1, 314-2, 440-1, 440-2, 504-1, 504-2, 504-3) comprises one or more of rubber and silicone.

14. The system of any of claims 1 to 13, wherein an elastic disc (551) is disposed on a flat-planar surface in the interior cavity.

15. The system of claim 14, wherein the elastic disc (551) is configured to seal with a top of the fluid container (216, 316, 416, 516).

## Patentansprüche

1. System zur Verwendung mit einer medizinischen Vorrichtung, wobei das System aufweist:
einen Flüssigkeitsbehälter (216, 316, 416, 516) auf, wobei der Flüssigkeitsbehälter (216, 316, 416, 516) einen Hals (224, 324, 424, 524) mit einem äußeren Gewindeabschnitt (208, 308, 408) aufweist, und
einen Flüssigkeitsbehälteradapter (100, 200, 300, 400, 500), der aufweist:
eine Hülse (210, 310, 410, 510) mit einem Innenhohlraum (212, 312, 412, 512), wobei der Innenhohlraum konfiguriert ist, mit dem Flüssigkeitsbehälter (216, 316, 416, 516) zu koppeln; und
ein elastisches Element (204, 314-1, 314-2, 440-1, 440-2, 504-1, 504-2, 504-3), das in dem Innenhohlraum angeordnet ist, wobei das elastische Element eine zylindrische Form aufweist und konfiguriert ist, sich mit dem äußeren Gewindeabschnitt (208, 308, 408) des Fluidbehälters (216, 316, 416, 516) zu verbinden.

2. System nach Anspruch 1, wobei das elastische Element (204, 314-1, 314-2, 440-1, 440-2, 504-1, 504-2, 504-3) konfiguriert ist, mit dem äußeren Abschnitt des Flüssigkeitsbehälters (216, 316, 416, 516) abzudichten.

3. System nach einem der Ansprüche 1 bis 2, wobei das elastische Element (314-1) einen ersten Abschnitt mit einer ersten Dicke und einen zweiten Abschnitt mit einer zweiten Dicke aufweist, wobei der zweite Abschnitt konfiguriert ist, sich mehr zu biegen als der erste Abschnitt.

4. System nach Anspruch 3, wobei die zweite Dicke geringer ist als die erste Dicke und/oder wobei der zweite Abschnitt ein einstellbares Gewinde aufweist.

5. System nach einem der Ansprüche 1 bis 4, wobei die Hülse steifer ist als das elastische Element.

6. System nach einem der Ansprüche 1 bis 5, wobei die Hülse ein oder mehrere Durchgangslöcher (109-1, 109-2, 209, 509) aufweist.

7. System nach Anspruch 6, wobei das eine oder die mehreren Durchgangslöcher (109-1, 109-2, 209, 509) konfiguriert sind, einen Schlauchsatz (106) in Fluidverbindung mit einem Innenabschnitt des Fluidbehälters anzuordnen.

8. System nach einem der Ansprüche 1 bis 7, das einen Spalt zwischen dem elastischen Element (204, 314-1, 314-2, 440-1, 440-2, 504-1, 504-2, 504-3) und einem Abschnitt der Hülse (210, 310, 410, 510) aufweist, wobei das elastische Element konfiguriert ist, sich in mindestens einen Teil des Spalts zu verschieben, wenn es mit dem äußeren Abschnitt des Flüssigkeitsbehälters in Kontakt kommt.

9. System nach einem der Ansprüche 1 bis 8, wobei das elastische Element (204, 314-1, 314-2, 440-1, 440-2, 504-1, 504-2, 504-3) ein Formgedächtnis-Elastomer aufweist.

10. System nach einem der Ansprüche 1 bis 9, wobei das elastische Element (204, 314-1, 314-2, 440-1, 440-2, 504-1, 504-2, 504-3) durch Überspritzen in dem Innenhohlraum (212, 312, 412, 512) angeordnet ist.

11. System nach einem der Ansprüche 1 bis 10, wobei die Hülle (510) eine oder mehrere Gleitschienen (550-1, 550-2, 550-3) mit mindestens einem Schlitten (552-1, 552-2, 552-3) aufweist, der in jeder der einen oder mehreren Gleitschienen (550-1, 550-2, 550-3) angeordnet ist.

12. System nach Anspruch 11, wobei mindestens ein Abschnitt des elastischen Elements auf jedem der einen oder mehreren Schlitten (552-1, 552-2, 552-3) angeordnet ist.

13. System nach einem der Ansprüche 1 bis 12, wobei das elastische Element (204, 314-1, 314-2, 440-1, 440-2, 504-1, 504-2, 504-3) eines oder mehreres von Gummi und Silikon aufweist.

14. System nach einem der Ansprüche 1 bis 13, wobei eine elastische Scheibe (551) auf einer flach-planaren Fläche im Innenhohlraum angeordnet ist.

15. System nach Anspruch 14, wobei die elastische Scheibe (551) konfiguriert ist, mit einer Oberseite des Flüssigkeitsbehälters (216, 316, 416, 516) abzudichten.

## Revendications

1. Système destiné à être utilisé avec un dispositif médical, le système comprenant :
un récipient pour fluide (216, 316, 416, 516), le récipient pour fluide (216, 316, 416, 516) comprenant un goulot (224, 324, 424, 524) avec une partie filetée extérieure (208, 308, 408), et
un adaptateur de récipient pour fluide (100, 200, 300, 400, 500), comprenant :
une gaine (210, 310, 410, 510) avec une cavité intérieure (212, 312, 412, 512), dans laquelle la cavité intérieure est configurée pour être couplée au récipient pour fluide (216, 316, 416, 516) ; et
un élément élastique (204, 314-1, 314-2, 440-1, 440-2, 504-1, 504-2, 504-3) disposé dans la cavité intérieure, l'élément élastique comprenant une forme cylindrique et étant configuré pour s'interfacer avec la partie filetée extérieure (208, 308, 408) du récipient pour fluide (216, 316, 416, 516).

2. Système selon la revendication 1, dans lequel l'élément élastique (204, 314-1, 314-2, 440-1, 440-2, 504-1, 504-2, 504-3) est configuré de façon à former une étanchéité avec la partie extérieure du récipient pour fluide (216, 316, 416, 516).

3. Système selon l'une quelconque des revendications 1 à 2, dans lequel l'élément élastique (314-1) comprend une première partie ayant une première épaisseur et une deuxième partie ayant une deuxième épaisseur, dans lequel la deuxième partie est configurée pour fléchir davantage que la première partie.

4. Système selon la revendication 3, dans lequel la deuxième épaisseur est inférieure à la première épaisseur, et/ou dans lequel la deuxième partie comprend un filetage ajustable.

5. Système selon l'une quelconque des revendications 1 à 4, dans lequel la gaine est plus rigide que l'élément élastique.

6. Système selon l'une quelconque des revendications 1 à 5, dans lequel la gaine comprend un ou plusieurs trous traversants (109-1, 109-2, 209, 509).

7. Système selon la revendication 6, dans lequel les un ou plusieurs trous traversants (109-1, 109-2, 209, 509) sont configurés de façon qu'un jeu de tubes (106) soit placé en communication de fluide avec une partie intérieure du récipient pour fluide.

8. Système selon l'une quelconque des revendications 1 à 7, comprenant un espace entre l'élément élastique (204, 314-1, 314-2, 440-1, 440-2, 504-1, 504-2, 504-3) et une partie de la gaine (210, 310, 410, 510), l'élément élastique étant configuré pour se déplacer dans au moins une partie de l'espace quand il est interfacé avec la partie extérieure du récipient pour fluide.

9. Système selon l'une quelconque des revendications 1 à 8, dans lequel l'élément élastique (204, 314-1, 314-2, 440-1, 440-2, 504-1, 504-2, 504-3) comprend un élastomère à mémoire de forme.

10. Système selon l'une quelconque des revendications 1 à 9, dans lequel l'élément élastique (204, 314-1, 314-2, 440-1, 440-2, 504-1, 504-2, 504-3) est disposé dans la cavité intérieure (212, 312, 412, 512) via un surmoulage.

11. Système selon l'une quelconque des revendications 1 à 10, dans lequel la gaine (510) comprend une ou plusieurs glissières (550-1, 550-2, 550-3) avec au moins un coulisseau (552-1, 552-2, 552-3) disposé dans chacune des une ou plusieurs glissières (550-1, 550-2, 550-3).

12. Système selon la revendication 11, dans lequel au moins une partie de l'élément élastique est disposée sur chacun des un ou plusieurs coulisseaux (552-1, 552-2, 552-3).

13. Système selon l'une quelconque des revendications 1 à 12, dans lequel l'élément élastique (204, 314-1, 314-2, 440-1, 440-2, 504-1, 504-2, 504-3) comprend un ou plusieurs parmi un caoutchouc et une silicone.

14. Système selon l'une quelconque des revendications 1 à 13, dans lequel un disque élastique (551) est disposé sur une surface plate et plane dans la cavité intérieure.

15. Système selon la revendication 14, dans lequel le disque élastique (551) est configuré de façon à former une étanchéité avec la partie supérieure du récipient pour fluide (216, 316, 416, 516).
